# EUROPEAN PATENT SPECIFICATION

(11) **EP 4 346 572 B1**
(45) Date of publication and mention of the grant of the patent: **01.04.2026**
(21) Application number: 21749361.8
(22) Date of filing: 28.05.2021
(51) Int. Cl.: A61B 5/024, A61B 5/352

(54) **METHOD AND SYSTEM FOR QUALITY EVALUATION OF A SEQUENCE OF HEART INTERBEAT INTERVAL SIGNALS**
VERFAHREN UND SYSTEM ZUR QUALITÄTSBEURTEILUNG EINER SEQUENZ VON HERZ-ZWISCHENSCHLAG-INTERVALLSIGNALEN
PROCÉDÉ ET SYSTÈME D'ÉVALUATION DE QUALITÉ D'UNE SÉQUENCE DE SIGNAUX D'INTERVALLES D'INTERBATTEMENT CARDIAQUES

(43) Date of publication of application: 10.04.2024
(73) Proprietor: Harman International Industries, Incorporated, Stamford, CT 06901 (US)
(72) Inventor: SHISHALOV, Ivan Sergeevich, Nizhniy Novgorod, 603074 (RU); SHISHANOV, Sergey Valeryevich, Nizhny Novgorod, 603003 (RU); BAKHCHINA, Anastasiya Vladimirovna, Nizhniy Novgorod, 603069 (RU); KONOVALOV, Daniil Igorevich, Nizhny Novgorod, 603106 (RU); ERSHOV, Roman Aleksandrovich, Nizhny Novgorod, 603079 (RU); SHILOV, Andrey Sergeevich, Nizhny Novgorod, 603003 (RU); FILIMONOV, Andrey Viktorovich, Kamenki, 607610 (RU)
(74) Representative: Rummler, Felix
(86) International application number: PCT/RU2021/000225
(87) International publication number: WO 2022/250561

(56) References cited:
- EP-A1- 3 643 227
- WO-A1-2020/110116
- US-A1- 2007 021 675

## Description

### Field

The present disclosure relates to methods and systems for monitoring the health of a person, in particular to evaluate the quality of heart interbeat interval signals.

### Background

Heart beat features are a source of human physiological and psychological information. Parameters like heart interbeat intervals and its secondary measures heart rate and heart rate variability are essential to monitor a persons health and wellbeing. Contact heart rate measurement devices and sensors, such as pulsometers, ECG devices or PPG devices like smartwatches and wristbands, are widespread. There are also contactless devices available exploiting RGB cameras or radars as sensors (Harford et al., Physiol. Measurements, 40(6), 2019). In order to extract biosignals such as heart interbeat intervals from time-series signals generated from video images, remote photoplethysmography (rPPG) is often used. Contactless technologies are particularly appropriate for applications in e.g. in-cabin car monitoring systems. As modern cars are no longer just a means of commuting, but act as a living space, cars are integrated with more and more features and functions, sometimes not related to driving e.g. monitoring drivers and occupants physiological state and health. This is of particular use as cognitive load and stress, as well as (non-visual) distractions are known to strongly influence driving performance.

Thus, methods are being developed to process rPPG data containing physiological signals and enable the extraction of information for providing high quality datasets from rPPG measurements. For example, US 20160228069A1 discloses a method for processing physiological signals by determining and comparing (average) waveforms. US 8977347B2 discloses an rPPG method and system for estimating heart rate variability by extracting low frequency and high frequency components from a power spectrum of a time-series signal obtained by processing a video of the subject being monitored. Finzgar and Podrzaj (PeerJ. 2018, 6, e5859) describe a wavelet-based decomposition method for a robust extraction of pulse rate from video recordings. Wang et al. (IEEE Transa. Biomed. Engin. 99, 99, 2016) developed algorithmic principles of remote PPG and Wang, Stujik and de Haan (IEEE Transa. Biomed. Engin. 2015, DOI 10.1109/TBME.2015.2508602) focused their work on spatial subspace rotation in rPPG. Above methods aim to improve data acquisition using rPPG or biosignal extraction from raw data.

EP 3 643 227 A1 describes a method for estimating a user's minimum heart rate (min HR) from collected heart rate data. It includes calculating heart rate (HR) and artifact percentage from heartbeat signals over various time periods. Data is qualified if the user is verified to be awake and immobile, and the artifact percentage is below a set threshold; otherwise, the data is disqualified. The method then calculates heart rate parameters from the qualified data and applies a function to these parameters to approximate the min HR.

US 2007/021675 A1 describes a method and device for evaluating the entrainment between biological systems like heart rhythms and respiration using heart rate variability (HRV) and its power spectrum. Entrainment indicates a balanced autonomic nervous system, promoting health and performance. The method calculates an entrainment parameter (EP) from the power distribution in the HRV spectrum, with high EP values indicating concentrated power within a narrow frequency range. An apparatus for monitoring heartbeats and displaying this data on various devices is also described.

WO 2020/110116 A1 describes a system that monitors biological parameters of an individual using a coherent light source to illuminate a selected body region. It includes a light collection unit with an imaging unit that captures returning light to generate image data sequences. A control unit with a processor receives and processes this data to determine contrast variations, providing information about the individual's biological parameters.

The present disclosure provides a method and system for the selection of individual heart interbeat interval signals from an acquired signal sequence. When contactless biosignal measurement techniques are applied in non-medical environments, individual signals or subsequences of signals may by crucially deteriorated by disturbances in the measurement environment or contain outliers or measurement artifacts. The disclosed method allows eliminating individual signals of poor quality and selecting only remaining suitable signals for further data processing. This preselection enables a more accurate biosignal extraction due to high quality input data and more time- and computing power-efficient further data processing.

### Summary

The present invention is defined in the independent claims. The dependent claims recite selected optional features. In the following, each of the described methods, apparatuses, examples, and aspects, which do not fully correspond to the invention as defined in the claims is thus not according to the invention and is, as well as the whole following description, present for illustration purposes only or to highlight specific aspects or features of the claims.

A first aspect of the present disclosure relates to a method for selecting one or more heart interbeat interval, IBI, signals. The method comprises:
capturing a sequence of individual heart IBI signals using one or more sensors;
comparing the value of a first parameter of one of the individual heart IBI signals with a predetermined first threshold;
determining the value of a second parameter from one or more or all of the individual heart IBI signals of the sequence;
comparing the value of the second parameter of the one individual heart IBI signal with the value of the second parameter determined from one or more or all of the individual heart IBI signals of the sequence; and
selecting the one individual heart IBI signal based on the comparisons.

The objective of the method is to select biosignals, in particular heart interbeat interval signals, of high quality from a set of acquired raw data. The signal quality may be evaluated with respect to, for example, physiological meaning, signal-to-noise ratio or measurement artifacts. The disclosure relates to biosignal measurements of a user performed in a non-medical, non-ideal environment, such as the cabin of a car. Standard monitoring and diagnostic methods designed for medical applications can be used in such environments, however, harsh mechanical noise or rapidly changing lighting conditions can interfere with the detection of heart IBI signals and deteriorate signal quality. In some cases, the signal quality can be so low that measurements must be discarded to avoid false results during signal evaluation. Thus, the selection of suitable heart IBI signals of sufficient quality is critical for their application in biosignal processing. The disclosed method comprises several steps to determine and compare properties of individual signals in order to select suitable heart IBI signals from a sequence of acquired signals.

According to an embodiment of the present invention, the method comprises repeating the steps of comparing the value of a first parameter, determining the value of a second parameter, comparing the value of the second parameter, and selecting the one individual heart IBI signal based on the comparisons for each of the individual heart IBI signals of the sequence.

This procedure allows for statistically relevant determination of heart IBI signal characteristics and therefore statistically relevant data selection. Thereby, the complete data set may be cleared from signals of low quality.

In an embodiment, capturing the sequence of individual heart IBI signals is performed using a non-medical, contactless sensor. Using contactless sensing devices such as IR or visible light cameras or radar systems instead of conventional contact sensors commonly used for instance in smartwatches or other wearables opens up a new range of applications in non-ideal environments such as in-cabin user monitoring in a car.

In another embodiment, the first and the second parameter each comprise one or more of heart IBI RR interval, normal-to-normal interval, total power, standard deviation of normal-to-normal- interval, power spectral density, in particular, a low frequency and high frequency density distribution, signal amplitude, signal to noise ratio or peak distance. These parameters resemble indicators of specific characteristics of the heart IBI signals. Determining and evaluating one or more of a multitude of signal characteristics allows selecting the most suitable set of data for a given purpose or given measurement environment. Further, most relevant signal characteristics for a desired biosignal analysis or a signal property which is most easily distinguishable from noise signals in a given environment can be chosen as parameter for signal selection.

According to an embodiment of the invention, comparing the value of a first parameter comprises comparing the one individual heart IBI signal to physiologically valid individual heart IBI signals of a database; and wherein selecting the one individual heart IBI signal based on the comparisons comprises:
selecting the one individual heart IBI signal for further use if the individual heart IBI signal is within a predetermined range of the physiologically valid individual heart IBI signals, based on the comparison to the database;
dismissing the one individual heart IBI signal for further use if the individual heart IBI signal is not within a predetermined range of the physiologically valid individual heart IBI signals, based on the comparison to the database.

The effect of this embodiment is that individual heart IBI signals exhibiting physiologically non-meaningful characteristics can be discarded before they are fed to subsequent data processing routines. Values which are within a physiologically meaningful range are selected for further analysis, while for instance values suffering from clear measurement artifacts are removed.

Further in an embodiment, comparing the value of the second parameter comprises determining the absolute difference between the value of the second parameter of the one of the individual heart IBI signals and the value of the second parameter of the subsequent individual heart IBI signal of the sequence; and
wherein selecting the one individual heart IBI signal based on the comparisons comprises:
selecting the one individual heart IBI signal for further use if the absolute difference between the value of the second parameter of the one individual heart IBI signal and the value of the second parameter of the subsequent individual heart IBI signal is below a predetermined second threshold;
dismissing the one individual heart IBI signal for further use if the absolute difference between the value of the second parameter of the one individual heart IBI signal and the value of the second parameter of the subsequent individual heart IBI signal is above a predetermined second threshold.

The absolute difference between characteristic values of subsequent heart IBI signals within the signal sequence can act as a measure to remove individual outliers. A threshold for an absolute difference between directly neighboring heart IBI signals may for example be an interval length of 50 ms or 200 ms, depending on the required data quality and measurement environment.

In another embodiment, comparing the value of the second parameter comprises determining the relative difference between the value of the second parameter of the one of the individual heart IBI signals and the value of the second parameter of the subsequent individual heart IBI signal of the sequence; and
wherein selecting the one individual heart IBI signal based on the comparisons comprises:
selecting the one individual heart IBI signal for further use if the relative difference between the value of the second parameter of the one individual heart IBI signal and the value of the second parameter of its subsequent individual heart IBI signal is below a predetermined third threshold;
dismissing the one individual heart IBI signal for further use if the relative difference between the value of the second parameter of the one individual heart IBI signal and the value of the second parameter of its subsequent individual heart IBI signal is above a predetermined third threshold.

The relative difference between characteristic values of subsequent heart IBI signals within the signal sequence can also act as a measure to remove individual outliers. A threshold for relative differences between directly neighboring heart IBI signals may for example be a difference by -0.5-+0-5, or preferable by -0.2-+0.4, depending on the required data quality.

According to another embodiment of the invention, determining the value of a second parameter comprises determining an average of the values of the second parameter of two or more or all individual heart IBI signals of the sequence. This allows for a long-range data analysis, compared to the short-range comparison using absolute and relative differences to neighboring signals described above. Thereby, trends in signal characteristics occurring over a longer time span can be determined and taken into account during further signal selection.

Further, in an embodiment, comparing the value of the second parameter comprises determining a deviation of the value of the second parameter of the one individual heart IBI signal from the average of the values of the second parameter of two or more or all individual heart IBI signal of the sequence; and
wherein selecting the one individual heart IBI signal based on the comparisons comprises:
selecting the one individual heart IBI signal for further use if the deviation of the value of the second parameter of the one individual heart IBI signal from the average of the value of the second parameter of two or more or all individual heart IBI signal of the sequence is below a predetermined forth threshold;
dismissing the one individual heart IBI signal for further use if the deviation of the value of the second parameter of the one individual heart IBI signal from the average of the value of the second parameter of two or more or all individual heart IBI signal of the sequence is above a predetermined forth threshold.

In contrast to selecting individual heart IBI signals based on absolute and relative differences to their direct neighbors, it may be useful to compare characteristic values of individual signals to the average value of a sequence of signals. Depending on the desired quality of the selected set of heart IBI signals for a particular application, the sequence of signals from which an average value is determined may comprise few signals, for example five heart IBI signals, or more signals, for example hundreds or thousands of signals. Trends within signal evolution along longer signal sequences may be considered during signal selection, such as constantly increasing heart IBI shortening, which stays undetected when comparing only neighboring signals.

Selecting the one individual heart IBI signal based on the comparisons further comprises, according to an embodiment dismissing one or more selected individual heart IBI signals if the preceding individual heart IBI signal was dismissed and the two direct neighboring individual heart IBI signals of the preceding individual heart IBI signal were selected. Uncertainties in detecting whether an initially dismissed heart IBI signals or their subsequent signal is invalid can be overcome by invalidating both signals. Further, gaps in between evaluated short signal sequences or subsequences, defined by time windows or a number of IBIs, are not missed if such extension rules, as disclosed in this embodiment, are used. Further, this embodiment of the disclosed method prevents inserting data processing artifacts.

According to the invention, selecting the one individual heart IBI signal based on the comparisons further comprises dismissing one or more groups of selected individual heart IBI signals if the one or more groups of individual heart IBI signals are preceded and followed by groups of dismissed individual heart IBI signals, wherein a group of individual heart IBI signals comprises one ore more individual heart IBI signals.

If a small group of selected IBI signals is directly framed by two small groups of dismissed IBI signals, it is likely that measurement artifacts led to this situation. It is probable that the group of selected signals does not result from a physiologically meaningful data acquisition. Thus, the framed small group of selected signals is dismissed. A small group of signals may comprise for example two signals to tens of signals. A small group of signals may also comprise a single selected signal framed by single dismissed signals.

According to another embodiment, the method further comprises:
extracting one or more subsequences of individual heart IBI signals from the sequence of individual heart IBI signals;
performing any of the preceding methods on the one or more subsequences individually.

The subsequences may contain all individual heart IBI signals from a time window of 5 min, or preferable from 100 sec. Evaluating only a part of the whole acquired sequence enables dismissing subsequences which are invalid due to measurement circumstances, e.g. based on the measurement environment or user situation, prior to further data processing. For example, whole subsequences acquired during a time window in which a car, in which the method is performed, was exposed to significant mechanical noise can be dismissed. In another example, subsequences acquired in time windows wherein the user was in an unusual mental or physiological state, such as a state of mental distraction or during a cardiovascular emergency, may be evaluated independent from other subsequences. Performing any embodiment of the disclosed method only to one ore more selected relevant subsequences allows for more efficient and faster data analysis.

The invention further comprises, according to an embodiment:
extracting a subsequence of individual heart IBI signals from the sequence of individual heart IBI signals;
selecting the subsequences of individual heart IBI signals for further use if the number of dismissed individual heart IBI signals is below a predetermined fifth threshold;
dismissing the subsequences of individual heart IBI signals for further use if the number of dismissed individual heart IBI signals is above a predetermined fifth threshold.

Whole subsequences of the whole acquired heart IBI signal sequence may suffer from poor signal quality due to measurement artifacts in a particular measurement situation. For example, when performing the disclosed method in a non-ideal environment such as in a cabin of a car, a whole subsequence comprising a large number of individual signals may suffer from poor signal-to-noise ratio due to poor road conditions which result in high mechanical noise. Such subsequences may be dismissed completely during signal selection allowing for efficient and quick data selection.

According to a further embodiment, the first, second, third, forth and fifth threshold depend on the desired quality of the set of selected individual heart IBI signals.

Choosing the threshold values for signal selection is accompanied by a trade-off between signal quality and reliability. The stricter the rules and narrower the thresholds the better the quality, but less signals contribute to further analyses, which may often be required to be statistically relevant. Thus, it is beneficial to adjust the threshold according to the desired quality of the selected data set.

According to a second aspect of this disclosure, a system for selecting one or more heart interbeat interval signals is provided. The system comprises a sensor for acquiring one or more individual heart interbeat interval signals; and a computing device, wherein the system is configured to execute the steps described above. The sensor may be a non-medical, contactless sensor. The system may be installed in a car. All properties of the method of the present disclosure also apply to the system.

### Brief description of the drawings

The features, objects, and advantages of the present disclosure will become more apparent from the detailed description set forth below when taken in conjunction with the drawings in which like reference numerals refer to similar elements.
**Figure 1** depicts a flow chart of a method 100 for determining the quality of heart interbeat interval signals according to an embodiment;
**Figure 2** depicts a flow chart of a method 200 for determining the quality of individual heart interbeat interval signals using physiological values for comparison according to another embodiment;
**Figure 3** depicts a flow chart of a method 300 for determining the quality of individual heart interbeat interval signals using relative and absolute differences and average values according to an embodiment;
**Figure 4** depicts a schematic of a method 400 for determining the quality of the one or more individual heart IBI signals comparing values of parameters of one or more individual heart IBI signals and their neighboring one or more individual heart IBI signals according to an embodiment;
**Figure 5** depicts a block diagram of a system 500 for determining the quality of heart interbeat interval signals.

### Reference signs

100 Method for determining the quality of heart interbeat interval signals
102-110 Steps of method 100
200 Method for determining the quality of individual heart interbeat interval signals by comparison with physiological values
202-214 Steps of method 200
300 Method for determining the quality of individual heart interbeat interval signals using absolute and relative differences and averages
302-340 Steps of method 300
400 Method for determining the quality of the one or more individual heart IBI signals comparing values of parameters of one or more individual heart IBI signals and their neighboring one or more individual heart IBI signals
500 System for determining the quality of heart interbeat interval signals
502 Sensor
504 Computing device

### Detailed description of the preferred embodiments

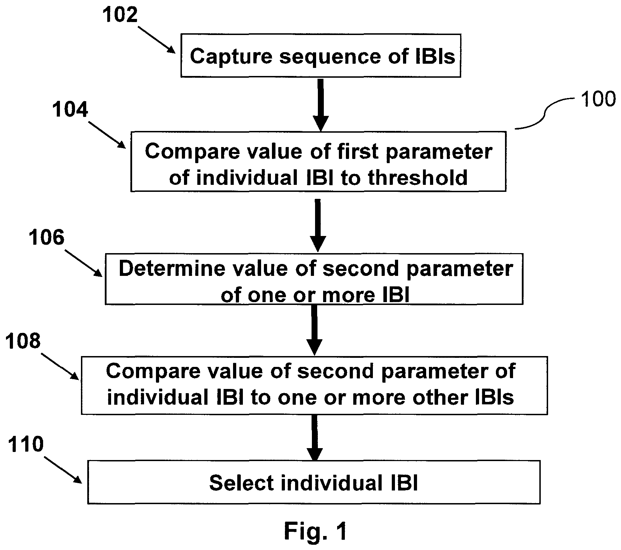

**Figure 1** depicts a flow chart of a method 100 for determining the quality of heart interbeat interval (IBI) signals according to an embodiment. First, a sequence of individual heart IBI signals is captured in step 102. According to an embodiment, the sequence is captured using a non-medical contactless sensor. For example, radar, IR or visible light images can be captured and heart IBI signals can be extracted using the remote PPG method. Using contactless sensors allows performing the method in a non-medical environment such as the cabin of a car. The value of a first parameter of individual heart IBI signals is then compared to a threshold in step 104. For example, an RR interval can be determined from the captured signals and compared to physiologically meaningful values. Further, the value of a second parameter of one or more heart IBI signals is determined in step 106. The first and the second parameter each comprise one or more of heart IBI RR interval, normal-to-normal interval, total power, standard deviation of normal-to-normal- interval, power spectral density, in particular, a low frequency and high frequency density distributions, signal amplitude, signal to noise ratio or peak distance. The first and second parameter may be the same or different.

For example, determining the value of the second parameter of one or more signals comprises determining values for each of the individual heart IBI signals or determining average values of two or more signals or determining the difference between subsequent signals. The value of the second parameter of one or more heart IBI signals is compared to the value of the second parameter of an individual heart IBI signal in step 108. For example, the value corresponding to an individual signal may be compared to an average value of two or more signals, or the value of an individual signal may be compared to the value of its preceding or following signal. In step 110, individual heart IBI signals are then selected according to the comparisons. For example, an individual heart IBI signal is selected, if the value of its first parameter is within a predetermined range of physiologically valid values. Or, for example, an individual heart IBI signal is selected, if the value of its second parameter differs from an average value by a predetermined percentage. The method 100 may be repeated for each of the individual heart IBI signals, or for a subsequence of the whole sequence of heart IBI signals or for the whole sequence. A subsequence can comprise a predetermined number of individual heart IBI features or all individual heart IBI signals from a predetermined time window. The time window may be 5 min or, preferably, 100 sec. Selected signals may be used for further data analysis, e.g. for determining second order biosignal metrics such as heart rate and heart rate variability.

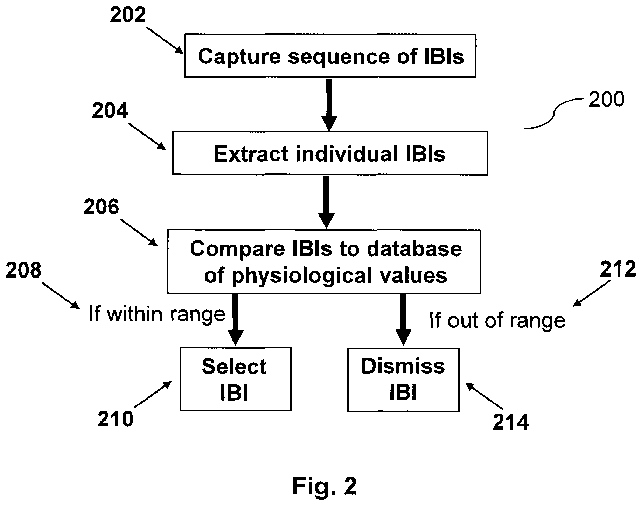

**Figure 2** depicts a flow chart of a method 200 for determining the quality of individual heart interbeat interval signals using physiological values for comparison according to another embodiment. A sequence of heart IBI signals is captured in step 202 and individual heart IBI signals are extracted from the sequence in step 204. In step 206, the values of the first parameter of the individual signals are compared to values in a database. The database may comprise ranges of physiologically meaningful values of the first parameter. For example, the database may comprise physiologically valid values for RR intervals. If the value of the individual heart IBI signal lies within this valid range (step 208), the individual signal is selected in step 210. If, however, the value of the individual signal lies out of this range (step 212), the individual signal is dismissed in step 214.

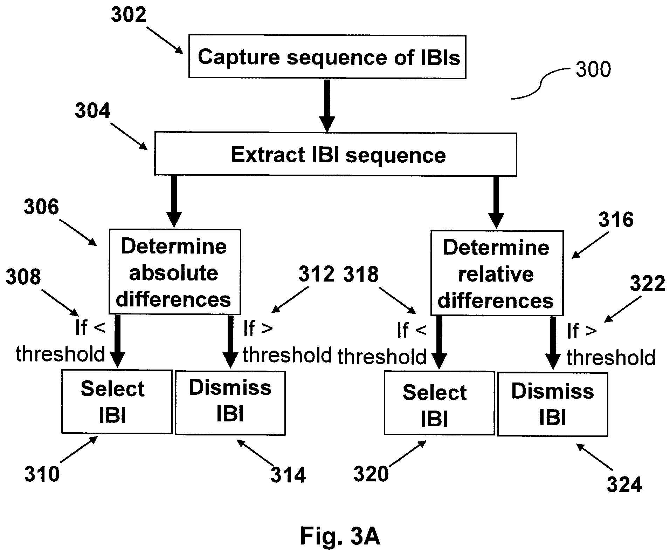

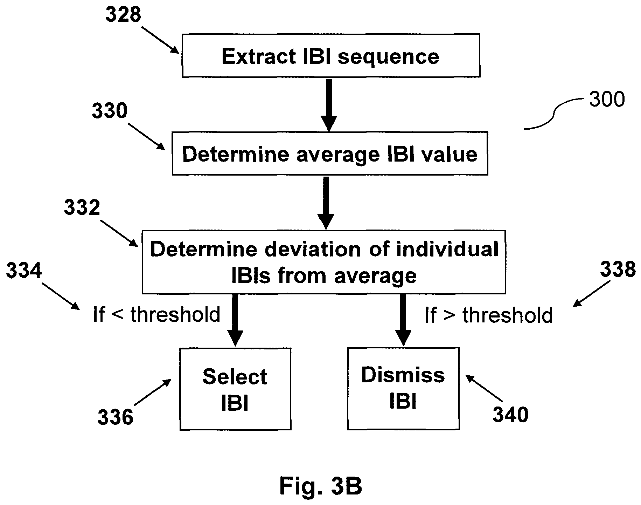

**Figure 3** depicts a flow chart of a method 300 for determining the quality of individual heart interbeat interval signals using relative and absolute differences and average values according to another embodiment. **Figure 3A** depicts an embodiment of the method 300 wherein a sequence of individual heart IBI signals is captured in step 302 and extracted in step 304. Either the complete sequence can be extracted or a subsequence can be extracted.

In steps 306 and 316 an absolute and a relative difference between subsequent individual heart IBI signals is determined, respectively. The absolute difference is determined by subtracting the value of the second parameter of an individual heart IBI signal n from the value of the second parameter of the n+1^{th} individual heart IBI signal. The relative difference is determined by dividing the absolute difference by the value of the n^{th} signal. If the absolute difference is below a predefined threshold (step 308), the signal is selected in step 310. If the absolute difference is above a predetermined threshold (step 312), the signal is dismissed in step 314. Analogous, if the relative difference is below a predetermined threshold (step 318), the signals is selected (step 320) or, if the relative difference is above a predetermined threshold (step 322), the signal is dismissed (step 324). In particular, for the signal to be selected in step 310, an absolute difference between RR intervals may be below 200 ms, or more particularly, below 50 ms. Further, in particular, the relative difference between RR intervals may be between -0.5 and +0.5, and more particularly, between -0.2 and +0.4 if the signal is to be selected in step 320. In an embodiment, both compared subsequent heart IBI signals (the n^{th} and n+ 1^{th} signal) are dismissed if their relative or absolute difference is above the predetermined thresholds. In another embodiment, heart IBI signals may only be selected if both, the relative and the absolute difference, are below a predetermined threshold, i.e. if the signals are selected in steps 310 and in step 320. **Figure 3B** depicts an embodiment of the method 300 wherein a heart IBI signal sequence or subsequence is extracted from the captured sequence in step 328 and an average value of the second parameter of the individual heart IBI signals of the sequence is determined in step 330. The average may be determined from a few signals, for example from five signals, or from a larger number of signals, for example from 100 signals. In step 332, the deviation of the value of the second parameter of an individual heart IBI signal from the average value is determined. If the deviation is below a predetermined threshold (334), the individual heart IBI signal is selected in step 336. If the deviation is above a predetermined threshold (338), the individual heart IBI signal is dismissed in step 340. One or more individual heart IBI signals from the extracted sequence may be dismissed.

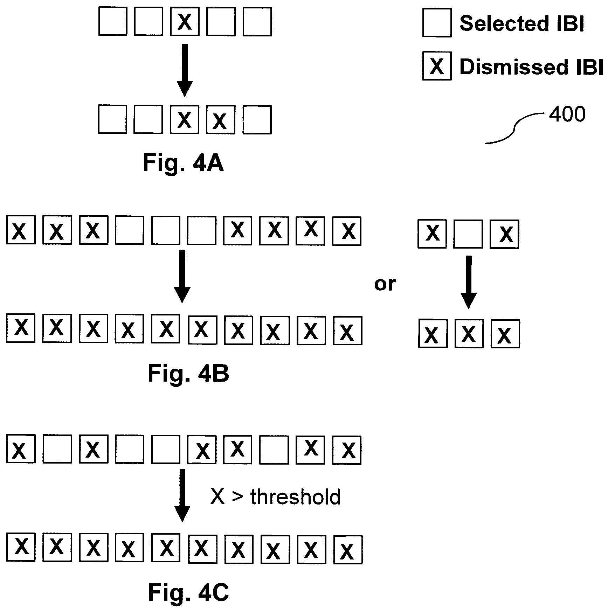

**Figure 4** depicts a schematic of rules of a method 400 for determining the quality of the one or more individual heart IBI signals comparing values of parameters of individual heart IBI signals and of their neighboring individual heart IBI signals according to an embodiment. **Figure 4A** depicts a schematic wherein a previously selected individual heart IBI signal is dismissed if its preceding individual heart IBI signal was dismissed and the two direct neighboring individual heart IBI signals of the preceding individual heart IBI signal were selected. **Figure 4B** exhibits a situation in which a group of selected individual heart IBI signals is dismissed if the group is preceded and followed by groups of dismissed individual heart IBI signals. A group of individual heart IBI signals may comprise few individual heart IBI signals, for example five signals. In an embodiment, the group may comprise one signal, i.e. an individual selected heart IBI signal is dismissed, if its both direct neighbors were dismissed. **Figure 4C** show a group or subsequence of individual heart IBI signals with randomly distributed dismissed signals. The whole group or subsequence is dismissed if said group or subsequence contains a number of dismissed individual heart IBI signals that is above a predetermined threshold.

In an embodiment, the methods or individual steps of the methods described above may be applied to individual heart IBI signals, a first subsequence of signals and a second subsequence of signals, wherein the second subsequence comprises more individual signals than the first subsequence, in subsequent data processing steps. This procedure minimizes the amount of dismissed individual heart IBI signals. Further in an embodiment, individual processing steps may be repeated for one or more times, skipped or the order of processing steps may be changed.

In an embodiment, extracted subsequences may comprise individual heart IBI signals from a time window of five minutes as it is common in and preferred for medical applications, or from a time window of 100 sec which is preferred for non-medical applications such as automotive applications. Example thresholds for possible first and second parameters are given below. Parameters including RR intervals (Interval between R-peaks in ECG), standard deviation of normal-to-normal intervals (SDNN), total power spectral density (TP), density distribution at low frequency (LF) and high frequency (HF), and sample entropy (SampEn), i.e. the likelihood of the occurrence of regularly repeating patterns on a scale from 0 (highly regular pattern) to 100 (highest entropy). All said parameters may be extracted from ECG measurements (medical application) or image series using rPPG (non-medical application).

| Parameter | Threshold range (for 5 min sequence) | Threshold range (for 100 sec sequence) |
|---|---|---|
| RR_mean | 460-1700 ms | 460-1700 ms |
| SDNN | 141 ± 39 ms | 10-150 ms |
| TP | 3466 ± 1018 ms² | 400-12000 ms² |
| LF | 1170 ± 416 ms² | 150-2100 ms² |
| HF | 975 ± 203 ms² | 120-2000 ms² |
| LF/HF | 1.5-2.0 | 0.2-1.7 |
| SampEn | - | 0.2-1.2 |

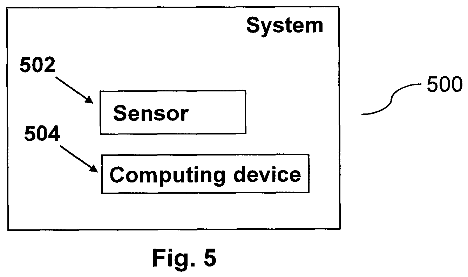

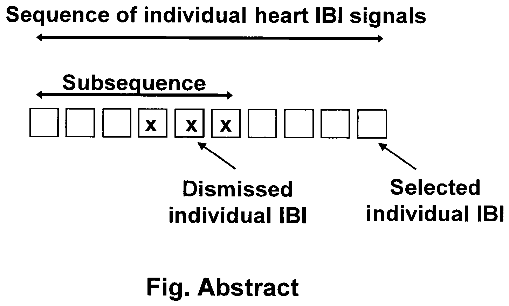

**Figure 5** depicts a block diagram of a system 500 for determining the quality of heart interbeat interval signals. The system 500 comprises a sensor 502, which is suitable for measuring heart interbeat interval signals. For example, the sensor 502 may comprise contact sensors such are ECG devices, pulsometers, smartwatches and wrist bands, or, preferably, contactless sensors, such as IR or visible light cameras used for rPPG. The system 500 may also comprise a plurality of sensors 502 from which the heart IBI signals are extracted. The system 500 further comprises a computing device 504 which is configured to execute the methods of all above embodiments. The system 500 may be installed in a non-medical environment such as inside the cabin of a car.

## Claims

1. A method (100) for selecting one or more heart interbeat interval, IBI, signals, the method comprising:
capturing a sequence of individual heart IBI signals (102) using one or more sensors (502);
comparing the value of a first parameter of one of the individual heart IBI signals with a predetermined first threshold (104);
determining the value of a second parameter from one or more or all of the individual heart IBI signals of the sequence (106);
comparing the value of the second parameter of the one individual heart IBI signal with the value of the second parameter determined from one or more or all of the individual heart IBI signals of the sequence (108); and
selecting the one individual heart IBI signal based on the comparisons (110), **characterized in that** selecting the one individual heart IBI signal based on the comparisons further comprises:
dismissing one or more selected individual heart IBI signals if the preceding individual heart IBI signal was dismissed and the two direct neighboring individual heart IBI signals of the preceding individual heart IBI signal were selected, and/or
dismissing one or more groups of selected individual heart IBI signals if the one or more groups of individual heart IBI signals are preceded and followed by groups of dismissed individual heart IBI signals, wherein a group of individual heart IBI signals comprises one or more individual heart IBI signals.

2. The method of claim 1, comprising repeating the steps of comparing the value of a first parameter, determining the value of a second parameter, comparing the value of the second parameter, and selecting the one individual heart IBI signal based on the comparisons for each of the individual heart IBI signals of the sequence.

3. The method of claim 1 or 2, wherein capturing the sequence of individual heart IBI signals (102) is performed using a non-medical, contactless sensor (502).

4. The method of any preceding claim, wherein the first and the second parameter each comprise one or more of heart IBI RR interval, normal-to-normal interval, total power, standard deviation of normal-to-normal- interval, power spectral density, in particular, a low frequency and high frequency density distribution, signal amplitude, signal to noise ratio or peak distance.

5. The method of any preceding claim, wherein comparing the value of a first parameter (104) comprises comparing the one individual heart IBI signal to physiologically valid individual heart IBI signals of a database; and
wherein selecting the one individual heart IBI signal based on the comparisons (110) comprises:
selecting the one individual heart IBI signal for further use if the individual heart IBI signal is within a predetermined range of the physiologically valid individual heart IBI signals, based on the comparison to the database;
dismissing the one individual heart IBI signal for further use if the individual heart IBI signal is not within a predetermined range of the physiologically valid individual heart IBI signals, based on the comparison to the database.

6. The method of any preceding claim, wherein comparing the value of the second parameter (108) comprises determining the absolute difference between the value of the second parameter of the one of the individual heart IBI signals and the value of the second parameter of the subsequent individual heart IBI signal of the sequence; and
wherein selecting the one individual heart IBI signal based on the comparisons comprises (110):
selecting the one individual heart IBI signal for further use if the absolute difference between the value of the second parameter of the one individual heart IBI signal and the value of the second parameter of the subsequent individual heart IBI signal is below a predetermined second threshold, in particular, if the absolute difference is below 200 ms, and more particularly, if the absolute difference is below 50 ms;
dismissing the one individual heart IBI signal for further use if the absolute difference between the value of the second parameter of the one individual heart IBI signal and the value of the second parameter of the subsequent individual heart IBI signal is above a predetermined second threshold.

7. The method of any preceding claim, wherein comparing the value of the second parameter (108) comprises determining the relative difference between the value of the second parameter of the one of the individual heart IBI signals and the value of the second parameter of the subsequent individual heart IBI signal of the sequence; and
wherein selecting the one individual heart IBI signal based on the comparisons (110) comprises:
selecting the one individual heart IBI signal for further use if the relative difference between the value of the second parameter of the one individual heart IBI signal and the value of the second parameter of its subsequent individual heart IBI signal is below a predetermined third threshold, in particular, if the relative difference is between -0.5 and + 0.5, and more particularly, if the relative difference is between -0.2 and + 0.4;
dismissing the one individual heart IBI signal for further use if the relative difference between the value of the second parameter of the one individual heart IBI signal and the value of the second parameter of its subsequent individual heart IBI signal is above a predetermined third threshold.

8. The method of any preceding claim, wherein determining the value of a second parameter (106) comprises determining an average of the values of the second parameter of two or more or all individual heart IBI signals of the sequence.

9. The method of any preceding claim, wherein comparing the value of the second parameter (108) comprises determining a deviation of the value of the second parameter of the one individual heart IBI signal from the average of the values of the second parameter of two or more or all individual heart IBI signal of the sequence; and
wherein selecting the one individual heart IBI signal based on the comparisons (110) comprises:
selecting the one individual heart IBI signal for further use if the deviation of the value of the second parameter of the one individual heart IBI signal from the average of the value of the second parameter of two or more or all individual heart IBI signal of the sequence, in particular from the average of five individual heart IBI signals, is below a predetermined forth threshold;
dismissing the one individual heart IBI signal for further use if the deviation of the value of the second parameter of the one individual heart IBI signal from the average of the value of the second parameter of two or more or all individual heart IBI signal of the sequence, in particular from the average of five individual heart IBI signals, is above a predetermined forth threshold.

10. The method of any of the preceding claims, further comprising
Extracting one or more subsequences of individual heart IBI signals from the sequence of individual heart IBI signals (204);
performing any of the preceding methods on the one or more subsequences individually,
wherein the subsequences preferably comprise individual heart IBI signals from a time window of 5 min, more preferably from a time window of 100 sec.

11. The method of any preceding claim, further comprising:
extracting a subsequence of individual heart IBI signals from the sequence of individual heart IBI signals (204);
selecting the subsequences of individual heart IBI signals for further use if the number of dismissed individual heart IBI signals is below a predetermined fifth threshold (210);
dismissing the subsequences of individual heart IBI signals for further use if the number of dismissed individual heart IBI signals is above a predetermined fifth threshold (214).

12. The method of any of the preceding claims, wherein the first, second, third, forth and fifth threshold depend on the desired quality of the set of selected individual heart IBI signals.

13. A system (500) for selecting one or more heart interbeat interval signals, the system comprising:
a sensor (502) for acquiring one or more individual heart interbeat interval signals; and
a computing device (504);
wherein:
the system (500) is configured to execute the method of any of claims 1 to 12.

## Patentansprüche

1. Verfahren (100) zum Auswählen eines oder mehrerer Herz-Zwischenschlag-Intervall(IBI)signale, wobei das Verfahren Folgendes umfasst:
Erfassen einer Sequenz individueller Herz-IBI-Signale (102) mithilfe eines oder mehrerer Sensoren (502);
Vergleichen des Wertes eines ersten Parameters eines der individuellen Herz-IBI-Signale mit einem vorbestimmten ersten Schwellenwert (104);
Bestimmen des Wertes eines zweiten Parameters aus einem oder mehreren oder allen individuellen Herz-IBI-Signalen der Sequenz (106);
Vergleichen des Wertes des zweiten Parameters des einen individuellen Herz-IBI-Signals mit dem Wert des zweiten Parameters, der aus einem oder mehreren oder allen individuellen Herz-IBI-Signalen der Sequenz (108) bestimmt wird; und
Auswählen des einen individuellen Herz-IBI-Signals basierend auf den Vergleichen (110), **dadurch gekennzeichnet, dass** das Auswählen des einen individuellen Herz-IBI-Signals basierend auf den Vergleichen ferner Folgendes umfasst:
Verwerfen eines oder mehrerer ausgewählter individueller Herz-IBI-Signale, wenn das vorhergehende individuelle Herz-IBI-Signal verworfen wurde und die zwei direkt benachbarten individuellen Herz-IBI-Signale des vorhergehenden individuellen Herz-IBI-Signals ausgewählt wurden, und/oder
Verwerfen einer oder mehrerer Gruppen ausgewählter individueller Herz-IBI-Signale, wenn der einen oder den mehreren Gruppen individueller Herz-IBI-Signale Gruppen verworfener individueller Herz-IBI-Signale vorausgehen und folgen, wobei eine Gruppe individueller Herz-IBI-Signale ein oder mehrere individuelle Herz-IBI-Signale umfasst.

2. Verfahren nach Anspruch 1, umfassend ein Wiederholen der Schritte des Vergleichens des Wertes eines ersten Parameters, des Bestimmens des Wertes eines zweiten Parameters, des Vergleichens des Wertes des zweiten Parameters und des Auswählens des einen individuellen Herz-IBI-Signals basierend auf den Vergleichen für jedes der individuellen Herz-IBI-Signale der Sequenz.

3. Verfahren nach Anspruch 1 oder 2, wobei das Erfassen der Sequenz individueller Herz-IBI-Signale (102) mithilfe eines nicht-medizinischen, kontaktlosen Sensors (502) durchgeführt wird.

4. Verfahren nach einem der vorhergehenden Ansprüche, wobei der erste und der zweite Parameter jeweils einen oder mehrere von einem Herz-IBI-RR-Intervall, einem Normal-zu-Normal-Intervall, einer Gesamtleistung, einer Standardabweichung des Normal-zu-Normal-Intervalls, einer Leistungsspektraldichte, insbesondere einer Niederfrequenz- und Hochfrequenz-Dichteverteilung, einer Signalamplitude, einem Signal-zu-Rausch-Verhältnis oder einem Spitzenabstand umfassen.

5. Verfahren nach einem der vorhergehenden Ansprüche, wobei das Vergleichen des Wertes eines ersten Parameters (104) ein Vergleichen des einen individuellen Herz-IBI-Signals mit physiologisch gültigen individuellen Herz-IBI-Signalen einer Datenbank umfasst; und
wobei das Auswählen des einen individuellen Herz-IBI-Signals basierend auf den Vergleichen (110) Folgendes umfasst:
Auswählen des einen individuellen Herz-IBI-Signals zur weiteren Verwendung, wenn das individuelle Herz-IBI-Signal innerhalb eines vorbestimmten Bereichs der physiologisch gültigen individuellen Herz-IBI-Signale ist, basierend auf dem Vergleich mit der Datenbank;
Verwerfen des einen individuellen Herz-IBI-Signals zur weiteren Verwendung, wenn das individuelle Herz-IBI-Signal nicht innerhalb eines vorbestimmten Bereichs der physiologisch gültigen individuellen Herz-IBI-Signale ist, basierend auf dem Vergleich mit der Datenbank.

6. Verfahren nach einem der vorhergehenden Ansprüche, wobei das Vergleichen des Wertes des zweiten Parameters (108) ein Bestimmen der absoluten Differenz zwischen dem Wert des zweiten Parameters des einen der individuellen Herz-IBI-Signale und dem Wert des zweiten Parameters des nachfolgenden individuellen Herz-IBI-Signals der Sequenz umfasst; und
wobei das Auswählen des einen individuellen Herz-IBI-Signals basierend auf den Vergleichen Folgendes umfasst (110):
Auswählen des einen individuellen Herz-IBI-Signals zur weiteren Verwendung, wenn die absolute Differenz zwischen dem Wert des zweiten Parameters des einen individuellen Herz-IBI-Signals und dem Wert des zweiten Parameters des nachfolgenden individuellen Herz-IBI-Signals unter einem vorbestimmten zweiten Schwellenwert ist, insbesondere, wenn die absolute Differenz unter 200 ms ist, und mehr insbesondere, wenn die absolute Differenz unter 50 ms ist;
Verwerfen des einen individuellen Herz-IBI-Signals zur weiteren Verwendung, wenn die absolute Differenz zwischen dem Wert des zweiten Parameters des einen individuellen Herz-IBI-Signals und dem Wert des zweiten Parameters des nachfolgenden individuellen Herz-IBI-Signals über einem vorbestimmten zweiten Schwellenwert ist.

7. Verfahren nach einem der vorhergehenden Ansprüche, wobei das Vergleichen des Wertes des zweiten Parameters (108) ein Bestimmen der relativen Differenz zwischen dem Wert des zweiten Parameters des einen der individuellen Herz-IBI-Signale und dem Wert des zweiten Parameters des nachfolgenden individuellen Herz-IBI-Signals der Sequenz umfasst; und
wobei das Auswählen des einen individuellen Herz-IBI-Signals basierend auf den Vergleichen (110) Folgendes umfasst:
Auswählen des einen individuellen Herz-IBI-Signals zur weiteren Verwendung, wenn die relative Differenz zwischen dem Wert des zweiten Parameters des einen individuellen Herz-IBI-Signals und dem Wert des zweiten Parameters seines nachfolgenden individuellen Herz-IBI-Signals unter einem vorbestimmten dritten Schwellenwert ist, insbesondere, wenn die relative Differenz zwischen -0,5 und +0,5 ist, und mehr insbesondere, wenn die relative Differenz zwischen -0,2 und +0,4 ist;
Verwerfen des einen individuellen Herz-IBI-Signals zur weiteren Verwendung, wenn die relative Differenz zwischen dem Wert des zweiten Parameters des einen individuellen Herz-IBI-Signals und dem Wert des zweiten Parameters seines nachfolgenden individuellen Herz-IBI-Signals über einem vorbestimmten dritten Schwellenwert ist.

8. Verfahren nach einem der vorhergehenden Ansprüche, wobei das Bestimmen des Wertes eines zweiten Parameters (106) ein Bestimmen eines Durchschnitts der Werte des zweiten Parameters von zwei oder mehr oder allen individuellen Herz-IBI-Signalen der Sequenz umfasst.

9. Verfahren nach einem der vorhergehenden Ansprüche, wobei das Vergleichen des Wertes des zweiten Parameters (108) ein Bestimmen einer Abweichung des Wertes des zweiten Parameters des einen individuellen Herz-IBI-Signals von dem Durchschnitt der Werte des zweiten Parameters von zwei oder mehr oder allen individuellen Herz-IBI-Signalen der Sequenz umfasst; und
wobei das Auswählen des einen individuellen Herz-IBI-Signals basierend auf den Vergleichen (110) Folgendes umfasst:
Auswählen des einen individuellen Herz-IBI-Signals zur weiteren Verwendung, wenn die Abweichung des Wertes des zweiten Parameters des einen individuellen Herz-IBI-Signals von dem Mittelwert des Wertes des zweiten Parameters von zwei oder mehr oder allen individuellen Herz-IBI-Signalen der Sequenz, insbesondere von dem Mittelwert von fünf individuellen Herz-IBI-Signalen, unter einem vorbestimmten vierten Schwellenwert ist;
Verwerfen des einen individuellen Herz-IBI-Signals zur weiteren Verwendung, wenn die Abweichung des Wertes des zweiten Parameters des einen individuellen Herz-IBI-Signals von dem Mittelwert des Wertes des zweiten Parameters von zwei oder mehr oder allen individuellen Herz-IBI-Signalen der Sequenz, insbesondere von dem Mittelwert von fünf individuellen Herz-IBI-Signalen, über einem vorbestimmten vierten Schwellenwert ist.

10. Verfahren nach einem der vorhergehenden Ansprüche, ferner umfassend
Extrahieren einer oder mehrerer Teilsequenzen individueller Herz-IBI-Signale aus der Sequenz individueller Herz-IBI-Signale (204);
individuelles Durchführen eines der vorhergehenden Verfahren an der einen oder den mehreren Teilsequenzen,
wobei die Teilsequenzen vorzugsweise individuelle Herz-IBI-Signale aus einem Zeitfenster von 5 min, mehr vorzugsweise aus einem Zeitfenster von 100 s, umfassen.

11. Verfahren nach einem der vorhergehenden Ansprüche, ferner umfassend:
Extrahieren einer Teilsequenz individueller Herz-IBI-Signale aus der Sequenz individueller Herz-IBI-Signale (204);
Auswählen der Teilsequenzen individueller Herz-IBI-Signale zur weiteren Verwendung, wenn die Anzahl verworfener individueller Herz-IBI-Signale unter einem vorbestimmten fünften Schwellenwert (210) ist;
Verwerfen der Teilsequenzen individueller Herz-IBI-Signale zur weiteren Verwendung, wenn die Anzahl verworfener individueller Herz-IBI-Signale über einem vorbestimmten fünften Schwellenwert (214) ist.

12. Verfahren nach einem der vorhergehenden Ansprüche, wobei der erste, zweite, dritte, vierte und fünfte Schwellenwert von der gewünschten Qualität des Satzes ausgewählter individueller Herz-IBI-Signale abhängt.

13. System (500) zum Auswählen eines oder mehrerer Herz-Zwischenschlag-Intervallsignale, wobei das System Folgendes umfasst:
einen Sensor (502) zum Aufnehmen eines oder mehrerer individueller Herz-Zwischenschlag-Intervallsignale; und
eine Rechenvorrichtung (504);
wobei:
das System (500) konfiguriert ist, um das Verfahren nach einem der Ansprüche 1 bis 12 auszuführen.

## Revendications

1. Procédé (100) de sélection d'un ou de plusieurs signaux d'intervalles d'interbattement cardiaques, IBI, le procédé comprenant :
la capture d'une séquence de signaux IBI cardiaques individuels (102) à l'aide d'un ou de plusieurs capteurs (502) ;
la comparaison de la valeur d'un premier paramètre de l'un des signaux IBI cardiaques individuels avec un premier seuil prédéterminé (104) ;
la détermination de la valeur d'un second paramètre à partir d'un ou de plusieurs ou de la totalité des signaux IBI cardiaques individuels de la séquence (106) ;
la comparaison de la valeur du second paramètre du signal IBI cardiaque individuel avec la valeur du second paramètre déterminée à partir d'un ou de plusieurs ou de la totalité des signaux IBI cardiaques individuels de la séquence (108) ; et
la sélection du signal IBI cardiaque individuel sur la base des comparaisons (110), **caractérisé en ce que**
la sélection du signal IBI cardiaque individuel sur la base des comparaisons comprend également :
le rejet d'un ou de plusieurs signaux IBI cardiaques individuels sélectionnés si le signal IBI cardiaque individuel précédent a été rejeté et que les deux signaux IBI cardiaques individuels voisins directs du signal IBI cardiaque individuel précédent ont été sélectionnés, et/ou
le rejet d'un ou de plusieurs groupes de signaux IBI cardiaques individuels sélectionnés si les un ou plusieurs groupes de signaux IBI cardiaques individuels sont précédés et suivis de groupes de signaux IBI cardiaques individuels rejetés, dans lequel un groupe de signaux IBI cardiaques individuels comprend un ou plusieurs signaux IBI cardiaques individuels.

2. Procédé selon la revendication 1, comprenant la répétition des étapes de comparaison de la valeur d'un premier paramètre, de détermination de la valeur d'un second paramètre, de comparaison de la valeur du second paramètre et de sélection du signal IBI cardiaque individuel sur la base des comparaisons pour chacun des signaux IBI cardiaques individuels de la séquence.

3. Procédé selon la revendication 1 ou 2, dans lequel la capture de la séquence de signaux IBI cardiaques individuels (102) est réalisée à l'aide d'un capteur sans contact non médical (502).

4. Procédé selon une quelconque revendication précédente, dans lequel le premier et le second paramètre comprennent chacun un ou plusieurs des paramètres suivants : intervalle RR IBI cardiaque, intervalle normal à normal, puissance totale, écart type de l'intervalle normal à normal, densité spectrale de puissance, en particulier, une distribution de densité de basse fréquence et de haute fréquence, amplitude du signal, rapport signal sur bruit ou distance du pic.

5. Procédé selon une quelconque revendication précédente, dans lequel la comparaison de la valeur d'un premier paramètre (104) comprend la comparaison du signal IBI cardiaque individuel à des signaux IBI cardiaques individuels physiologiquement valides d'une base de données ; et
dans lequel la sélection du signal IBI cardiaque individuel sur la base des comparaisons (110) comprend :
la sélection du signal IBI cardiaque individuel pour une utilisation ultérieure si le signal IBI cardiaque individuel se situe dans une plage prédéterminée des signaux IBI cardiaques individuels physiologiquement valides, sur la base de la comparaison avec la base de données ;
le rejet du signal IBI cardiaque individuel pour une utilisation ultérieure si le signal IBI cardiaque individuel ne se situe pas dans une plage prédéterminée des signaux IBI cardiaques individuels physiologiquement valides, sur la base de la comparaison avec la base de données.

6. Procédé selon une quelconque revendication précédente, dans lequel la comparaison de la valeur du second paramètre (108) comprend la détermination de la différence absolue entre la valeur du second paramètre de l'un des signaux IBI cardiaques individuels et la valeur du second paramètre du signal IBI cardiaque individuel suivant de la séquence ; et
dans lequel la sélection du signal IBI cardiaque individuel sur la base des comparaisons comprend (110) :
la sélection du signal IBI cardiaque individuel pour une utilisation ultérieure si la différence absolue entre la valeur du second paramètre du signal IBI cardiaque individuel et la valeur du second paramètre du signal IBI cardiaque individuel suivant est inférieure à un deuxième seuil prédéterminé, en particulier, si la différence absolue est inférieure à 200 ms, et plus particulièrement, si la différence absolue est inférieure à 50 ms ;
le rejet du signal IBI cardiaque individuel pour une utilisation ultérieure si la différence absolue entre la valeur du second paramètre du signal IBI cardiaque individuel et la valeur du second paramètre du signal IBI cardiaque individuel suivant est supérieure à un deuxième seuil prédéterminé.

7. Procédé selon une quelconque revendication précédente, dans lequel la comparaison de la valeur du second paramètre (108) comprend la détermination de la différence relative entre la valeur du second paramètre de l'un des signaux IBI cardiaques individuels et la valeur du second paramètre du signal IBI cardiaque individuel suivant de la séquence ; et
dans lequel la sélection du signal IBI cardiaque individuel sur la base des comparaisons (110) comprend :
la sélection du signal IBI cardiaque individuel pour une utilisation ultérieure si la différence relative entre la valeur du second paramètre du signal IBI cardiaque individuel et la valeur du second paramètre de son signal IBI cardiaque individuel suivant est inférieure à un troisième seuil prédéterminé, en particulier, si la différence relative est comprise entre -0,5 et +0,5, et plus particulièrement, si la différence relative est comprise entre -0,2 et +0,4 ;
le rejet du signal IBI cardiaque individuel pour une utilisation ultérieure si la différence relative entre la valeur du second paramètre du signal IBI cardiaque individuel et la valeur du second paramètre de son signal IBI cardiaque individuel suivant est supérieure à un troisième seuil prédéterminé.

8. Procédé selon une quelconque revendication précédente, dans lequel la détermination de la valeur d'un second paramètre (106) comprend la détermination d'une moyenne des valeurs du second paramètre de deux signaux IBI cardiaques individuels ou plus, ou la totalité de ceux-ci, de la séquence.

9. Procédé selon une quelconque revendication précédente, dans lequel la comparaison de la valeur du second paramètre (108) comprend la détermination d'un écart de la valeur du second paramètre du signal IBI cardiaque individuel par rapport à la moyenne des valeurs du second paramètre de deux signaux IBI cardiaques individuels ou plus, ou la totalité de ceux-ci, de la séquence ; et
dans lequel la sélection du signal IBI cardiaque individuel sur la base des comparaisons (110) comprend :
la sélection du signal IBI cardiaque individuel pour une utilisation ultérieure si l'écart de la valeur du second paramètre du signal IBI cardiaque individuel par rapport à la moyenne de la valeur du second paramètre de deux signaux IBI cardiaques individuels ou plus, ou la totalité de ceux-ci, de la séquence, en particulier par rapport à la moyenne de cinq signaux IBI cardiaques individuels, est inférieur à un quatrième seuil prédéterminé ;
le rejet du signal IBI cardiaque individuel pour une utilisation ultérieure si l'écart de la valeur du second paramètre du signal IBI cardiaque individuel par rapport à la moyenne de la valeur du second paramètre de deux signaux IBI cardiaques individuels ou plus, ou la totalité de ceux-ci, de la séquence, en particulier par rapport à la moyenne de cinq signaux IBI cardiaques individuels, est supérieur à un quatrième seuil prédéterminé.

10. Procédé selon l'une quelconque des revendications précédentes, comprenant également
l'extraction d'une ou de plusieurs sous-séquences de signaux IBI cardiaques individuels à partir de la séquence de signaux IBI cardiaques individuels (204) ;
la réalisation de l'un quelconque des procédés précédents sur les une ou plusieurs sous-séquences individuellement,
dans lequel les sous-séquences comprennent de préférence des signaux IBI cardiaques individuels provenant d'une fenêtre temporelle de 5 minutes, de manière davantage préférée d'une fenêtre temporelle de 100 secondes.

11. Procédé selon une quelconque revendication précédente, comprenant également :
l'extraction d'une sous-séquence de signaux IBI cardiaques individuels à partir de la séquence de signaux IBI cardiaques individuels (204) ;
la sélection des sous-séquences de signaux IBI cardiaques individuels pour une utilisation ultérieure si le nombre de signaux IBI cardiaques individuels rejetés est inférieur à un cinquième seuil prédéterminé (210) ;
le rejet des sous-séquences de signaux IBI cardiaques individuels pour une utilisation ultérieure si le nombre de signaux IBI cardiaques individuels rejetés est supérieur à un cinquième seuil prédéterminé (214).

12. Procédé selon l'une quelconque des revendications précédentes, dans lequel les premier, deuxième, troisième, quatrième et cinquième seuils dépendent de la qualité souhaitée de l'ensemble de signaux IBI cardiaques individuels sélectionnés.

13. Système (500) de sélection d'un ou de plusieurs signaux d'intervalles d'interbattement cardiaques, le système comprenant :
un capteur (502) pour acquérir un ou plusieurs signaux d'intervalles d'interbattement cardiaques individuels ; et
un dispositif informatique (504) ;
dans lequel :
le système (500) est configuré pour exécuter le procédé selon l'une quelconque des revendications 1 à 12.
